# FASCICULE DE BREVET EUROPEEN

(11) **EP 1 878 401 B1**
(45) Date de publication et mention de la délivrance du brevet: **24.09.2008**
(21) Numéro de dépôt: 06014540.6
(22) Date de dépôt: 13.07.2006
(51) Int. Cl.: A61C 1/08, A61B 17/16

(54) **Pièce à main à usage dentaire ou chirurgical**
Dentalmedizinisches oder chirurgisches Handstück
Dental or surgical handpiece

(43) Date de publication de la demande: 16.01.2008
(73) Titulaire: Bien-Air Holding SA, 2500 Bienne 6 (CH)
(72) Inventeur: Juan, Alain, 2072 Saint-Blaise (CH)
(74) Mandataire: Ravenel, Thierry Gérard Louis

(56) Documents cités:
- WO-A-99/45161
- US-A1- 2005 191 597
- US-B1- 6 200 685

## Description

La présente invention concerne une pièce à main à usage dentaire ou chirurgical. Plus précisément, la présente invention concerne un tel instrument à usage dentaire ou chirurgical dont le corps est réalisé dans un matériau léger et facile à usiner.

Il existe principalement deux familles d'instruments à main à usage dentaire ou chirurgical. La première de ces familles regroupe ce que l'on appelle les turbines, à savoir des instruments dont l'outil, par exemple une fraise, est couplé à un moteur entraîné par de l'air sous pression. Un tel moteur peut tourner à plusieurs centaines de milliers de tours par minute avec une pression d'air typiquement de l'ordre de 2,5 à 3 bars. Le corps d'une telle turbine par lequel le praticien tient son instrument en main est classiquement relié à une unité d'alimentation électrique et de fourniture de fluides (air, eau) via un raccord et un tuyau souple. La seconde famille d'instruments à usage dentaire ou chirurgical regroupe ce que l'on appelle les pièces à main et les contre-angles. Les pièces à main et les contre-angles diffèrent pour l'essentiel en ce que le corps des pièces à main est sensiblement droit tandis que celui des contres-angles forme un angle en un endroit de sa longueur. Pour le reste, ces deux types d'instruments se ressemblent beaucoup. Un moteur, fixé sur la pièce à main ou le contre-angle et capable de tourner à plusieurs dizaines de milliers de tours par minute, est relié via un tuyau à raccord fixe ou tournant à l'unité d'alimentation.

Pour des raisons de commodité, on désignera dans ce qui suit les instruments à usage dentaire ou chirurgical tels que décrits ci-dessus par l'expression générique pièces à main, sachant que cette expression recouvre tout aussi bien les pièces à main proprement dites que les turbines et les contre-angles.

Les reproches les plus fréquemment adressés par les praticiens aux pièces à main qu'ils utilisent quotidiennement dans l'exercice de leur métier visent la résistance à l'usure et le poids de ces instruments. En effet, ces pièces à main sont le plus souvent réalisées en laiton ou en acier inoxydable (voir par exemple le document US 2005/0191597). Le laiton présente l'inconvénient de s'oxyder facilement et doit donc subir des traitements de surface par dépôts de couches de matériaux inoxydables. Ces couches, relativement fragiles, ont tendance à s'user et à se rayer facilement, pouvant laisser apparaître par endroits la couche de laiton sous-jacente, ce qui donne aux praticiens le sentiment d'un instrument de qualité médiocre. Quant à l'acier inoxydable, bien qu'il soit un matériau apprécié par les constructeurs de pièces à main pour ses qualités d'usinabilité, de dureté et de résistance à la corrosion, il présente l'inconvénient d'être très lourd. Ainsi, une pièce à main réalisée en acier inoxydable sera pesante et donc fatigante pour celui qui doit la tenir en main plusieurs heures chaque jour.

Des tentatives ont déjà été menées pour essayer de remédier aux problèmes de résistance à l'usure et de poids des pièces à main à usage dentaire ou chirurgical. Ainsi, il a déjà été proposé de réaliser le corps des pièces à main en titane. Ce matériau présente le double avantage d'être résistant et léger. Ce matériau est néanmoins difficile à usiner car il présente des problèmes d'auto-inflammabilité. En effet, pour réaliser une pièce à main en titane, on part d'un barreau réalisé dans ce matériau que l'on va ensuite percer, fraiser, fileter etc. pour que le corps de la pièce à main résultant réponde aux besoins de son constructeur. Or, on s'est aperçu qu'en raison des hautes vitesses auxquelles tournent les outils d'usinage, le titane avait tendance à s'enflammer spontanément ce qui, on le comprendra aisément, n'est pas acceptable dans un environnement industriel. Pour remédier à ce problème, il est possible d'usiner le barreau de titane dans une atmosphère exempte d'oxygène, ce qui est néanmoins contraignant et rend le travail de l'opérateur encore plus difficile. Par ailleurs, on s'est également rendu compte que lorsqu'on usine le titane, celui-ci a tendance à former des copeaux qui adhèrent aux outils d'usinage. Ainsi, il n'est pas rare de devoir réaliser plusieurs passes pour pouvoir percer un trou dans un barreau de titane, l'opérateur devant, entre deux passes successives, débarrasser l'outil de perçage des copeaux de titane qui se sont agglomérés autour dudit outil. Enfin, on a constaté que les pièces à main réalisées en titane présentaient des problèmes d'ordre esthétique, la couleur du titane étant très froide et peu plaisante à l'oeil.

La présente invention a pour but de remédier aux problèmes susmentionnés ainsi qu'à d'autres encore en procurant un instrument à usage dentaire ou chirurgical qui soit notamment léger et esthétique.

A cet effet, la présente invention concerne une pièce à main à usage dentaire ou chirurgical comprenant un corps par lequel le praticien tient l'instrument, caractérisée en ce que le corps est réalisé en un alliage de titane-molybdène.

Grâce à ces caractéristiques, la présente invention procure un instrument à usage dentaire ou chirurgical qui est réalisé en un matériau qui allie légèreté, résistance mécanique et usinabilité. On s'est en effet rendu compte que l'alliage de titane-molybdène était beaucoup plus facile à usiner que le titane pur. En particulier, lorsqu'on pratique des perçages, des filetages, des alésages ou autres dans un barreau d'alliage de titane-molybdène, la matière ôtée se désagrège sous forme de poudre plutôt que sous forme de copeaux comme tel est le cas avec un barreau de titane pur, ce qui simplifie considérablement les opérations d'usinage. Surtout, l'alliage de titane-molybdène ne s'enflamme pas lorsqu'il est usiné à l'aide d'outils tournant à haute vitesse, ce qui rend le travail de cette matière beaucoup plus sûr dans un environnement industriel.

L'alliage de titane-molybdène présente également une grande résistance mécanique tout en étant extrêmement léger, ce qui améliore sensiblement le confort d'utilisation d'une pièce à main à usage dentaire ou chirurgical réalisée dans un tel matériau. Un autre avantage déterminant de alliage de titane-molybdène réside dans le fait qu'il présente une haute résistance à la corrosion. Ceci s'avère déterminant dans le domaine dentaire et chirurgical où les instruments sont particulièrement agressés par les différents traitements de nettoyage, de désinfection et de stérilisation. On s'est également rendu compte que les corps de pièces à main réalisés en alliage de titane-molybdène présentaient une couleur blanche très chaude et agréable à l'oeil. Il est donc inutile de revêtir l'alliage de titane-molybdène de couches de traitement de surface additionnelles, un simple traitement mécanique de la surface de l'alliage, par exemple par micro-billage, étant suffisant pour éliminer les traits d'usinage et procurer un état de surface remarquable. Ceci permet donc de réaliser des économies substantielles mais surtout d'éviter les problèmes liés aux couches de traitement de surfaces additionnelles qui ont tendance à s'user rapidement. Enfin on notera que le titane-molybdène satisfait à toutes les exigences en termes de biocompatibilité.

Selon une caractéristique complémentaire de l'invention, l'alliage de titane-molybdène comprend environ 80% en poids de titane et le complément en poids de molybdène.

Il va de soi que la présente invention n'est pas limitée au mode de réalisation qui vient d'être décrit et que diverses modifications et variantes simples peuvent être envisagées par l'homme du métier sans sortir du cadre de l'invention tel que défini par les revendications annexées.

## Revendications

1. Pièce à main à usage dentaire ou chirurgical comprenant un corps par lequel le praticien tien l'instrument, **caractérisée en ce que** le corps est réalisé en un alliage de titane-molybdène.

2. Pièce à main selon la revendication 1, **caractérisée en ce que** l'alliage de titane-molybdène comprend environ 80% en poids de titane et le complément en poids de molybdène.

## Claims

1. Handpiece for dental or surgical use including a body via which the practitioner holds the instrument, **characterized in that** the body is made of a titanium-molybdenum alloy.

2. Handpiece according to claim 1, **characterized in that** the titanium-molybdenum alloy includes approximately 80% titanium by weight and the remaining weight in molybdenum.

## Patentansprüche

1. Dentalmedizinisches- oder chirurgisches Handstück, welches einen Körper umfasst, an dem der Bediener das Instrument hält, **dadurch gekennzeichnet, dass** der Körper aus einer Titan-Molybdän-Legierung hergestellt ist.

2. Handstück nach Anspruch 1, **dadurch gekennzeichnet, dass** die Titan-Molybdän-Legierung ungefähr 80 Gewichts -% Titan und das verbleibende Gewicht aus Molybdän umfasst.
